# EUROPÄISCHE PATENTSCHRIFT

(11) **EP 2 144 690 B1**
(45) Veröffentlichungstag und Bekanntmachung des Hinweises auf die Patenterteilung: **28.07.2010**
(21) Anmeldenummer: 08748984.5
(22) Anmeldetag: 18.04.2008
(51) Int. Cl.: B01D 53/62, B01D 53/73, B01D 53/75

(54) **VERFAHREN ZUM REINIGEN VON RAUCHGASEN AUS VERBRENNUNGSANLAGEN MIT ANSCHLIESSENDER HARNSTOFFERZEUGUNG**
METHOD FOR PURIFYING FLUE GASES FROM INCINERATORS AND THEN PRODUCING UREA
PROCÉDÉ D'ÉPURATION DES GAZ DE COMBUSTION D'INCINÉRATEURS ET PRODUCTION ULTÉRIEURE D'URÉE

(30) Priorität: 02.05.2007 DE 102007020855
(43) Veröffentlichungstag der Anmeldung: 20.01.2010
(73) Patentinhaber: Evonik Energy Services GmbH, 45128 Essen (DE)
(72) Erfinder: WINKLER, Hermann, 45665 Recklinghausen (DE)
(74) Vertreter: Zenz
(86) Internationale Anmeldenummer: PCT/EP2008/003129
(87) Internationale Veröffentlichungsnummer: WO 2008/135150

(56) Entgegenhaltungen:
- WO-A-2006/022885
- US-A- 3 037 844
- US-A- 4 434 144
- US-A- 5 832 712

## Beschreibung

Die vorliegende Erfindung betrifft ein Verfahren zum Reinigen von Rauchgasen aus Verbrennungsanlagen, insbesondere Kraftwerken. Insbesondere betrifft die vorliegende Erfindung ein Verfahren zur Entfernung von Kohlendioxid aus solchen Rauchgasen.

Abgase aus Verbrennungsanlagen, sogenannte Rauchgase, weisen eine Anzahl von Schadstoffen auf, die entsprechend den heutigen Umweltbestimmungen aus diesen entfernt werden müssen. Die zu entfernenden Schadstoffe umfassen beispielsweise Schwefeloxide, Stickoxide und anorganische Fluor- und Chlorverbindungen. Neue Verbrennungsanlagen sind daher mit Rauchgasreinigungsanlagen ausgerüstet, die in den Rauchgasen enthaltene Schwefeloxide, Stickoxide und anorganische Fluor- und Chlorverbindungen aus diesen entfernen. Üblicherweise werden die Rauchgase ferner durch eine Reihe von Filtern geleitet, um A-schepartikel aus den Rauchgasen zu entfernen.

In jüngster Zeit werden vermehrt Anstrengungen unternommen, auch zumindest einen Teil des Kohlendioxids aus den Rauchgasen zu entfernen, da es sich bei Kohlendioxid um ein sogenanntes Treibhausgas handelt, welches mit verantwortlich für den sogenannten Treibhauseffekt ist.

Ein Ansatz zur Verminderung der CO₂-Immission ist es, dieses aus dem Rauchgasstrom zu entfernen und tief in der Erde oder unter dem Meeresgrund zu speichern. Dieser Ansatz hat jedoch den Nachteil, dass nicht gewährleistet ist, dass durch tektonische Verschiebungen das derart eingelagerte Kohlendioxid nicht wieder freigesetzt wird. Ferner hat dieser Ansatz den Nachteil, dass mit ihm ausgesprochen hohe Kosten verbunden sind - zum einen für die Ortung geeigneter Lagerplätze, zum anderen für die eigentliche Einlagerung in den Lagerplätzen.

Aus der US 4,434,144 ist ferner ein Verfahren zum Entfernen von CO₂ und/oder H₂S aus Gasmischungen bekannt, bei welchem die Gasmischungen mit einer Absorptionslösung behandelt werden, wobei diese zwischen einer Absorptionsstufe und einer Regenerationsstufe zirkuliert wird. Die Absorptionslösung umfasst mehrere, die Absorption von CO₂ und/oder H₂S begünstigende Substanzen, sowie eine die Regeneration der Lösung begünstigende Substanz.

Die hohen, mit bekannten Verfahren zur Verminderung der CO₂-Immission verbundenen Kosten verhindern bzw. erschweren eine umfangreiche Nutzung dieser Verfahren.

Aufgabe der Erfindung ist es daher, ein alternatives, kostengünstiges Verfahren zum Entfernen von Kohlendioxid aus Rauchgasen bereitzustellen.

Diese Aufgabe wird gelöst durch ein Verfahren mit den Merkmalen von Patentanspruch 1.

Bei einem ersten Verfahrensschritt werden die Rauchgase entstaubt und entstickt, wobei die Reihenfolge dieser beiden Reinigungsschritte nicht wesentlich für die Erfindung ist. Das Entstauben im Sinne dieser Erfindung umfaßt das Entfernen von Feinasche, auch als Staub bezeichnet, sowie das Entfernen von Grobasche aus den Rauchgasen. Aus dem Stand der Technik sind zahlreiche Verfahren bzw. Vorrichtungen zum Entfernen von Staub bzw. Asche aus Rauchgasen bekannt. Diese umfassen Massenkraftabscheider, Filtrationsabscheider, Elektrofilter und Nassabscheider. Die vorliegende Erfindung ist nicht auf eins der vorgenannten Abscheideverfahren beschränkt - je nach Anwendungsgebiet des erfindungsgemäßen Verfahrens kann ein beliebiges Verfahren zum Entstauben verwendet werden.

Zum Entsticken der Rauchgase kann ein beliebiges aus dem Stand der Technik bekanntes Verfahren zur NOₓ-Abscheidung verwendet werden. Im Rahmen dieser Erfindung anwendbare Verfahren lassen sich folgendermaßen gliedern: a) selektive nichtkatalytische Reduktion (SNCR), b) selektive katalytische Reduktion (SCR), und c) andersartige trockene NOₓ-Abscheideverfahren (z.B. Elektronenstrahlverfahren (ESV)).

Bei einem nachfolgenden Verfahrensschritt wird in der Gegenwart eines Oxidationsmittels zumindest ein Teil der Rauchgase mit einer wäßrigen Ammoniaklösung in Kontakt gebracht. Bei diesem in Kontakt bringen reagiert das Kohlendioxid aus dem Rauchgas mit dem Ammoniak in der Lösung zu Ammoniumcarbonat, wobei eine Reaktionslösung entsteht, in der Ammoniumcarbonat gelöst vorliegt. Die Rauchgase enthalten ferner Schwefeldioxid, welches bei dem in Kontakt bringen der Rauchgase mit der wäßrigen Ammoniaklösung in Gegenwart eines Oxidationsmittels zu Ammoniumsulfat umgesetzt wird und nach seiner Bildung gelöst in der Reaktionslösung vorliegt. Als Oxidationsmittel wird üblicherweise Luftsauerstoff verwendet, jedoch können im Rahmen dieser Erfindung auch andere Oxidationsmittel verwendet werden. Für den Fall, dass die Rauchgase keine ausreichend hohe Konzentration Sauerstoff ausweisen, um eine Oxidation des Schwefeldioxids zu gewährleisten, kann zusätzliches Oxidationsmittel zugeführt werden, und zwar beispielsweise direkt in die Rauchgase vor Eintritt in die Wascheinrichtung, oder aber bei Inkontaktbringung mit der Ammoniaklösung.

Bei dem in Kontakt bringen der Rauchgase mit der wäßrigen Ammoniaklösung gelingt es somit, die Konzentration von zwei Gasen in den Rauchgasen zu vermindern, nämlich die Konzentration von Kohlendioxid sowie die Konzentration von Schwefeldioxid. Das in Kontakt bringen selbst kann beispielsweise durch ein Einsprühen der wäßrigen Ammoniaklösung erfolgen. In Abhängigkeit von dem Volumenstrom sind jedoch auch zahlreiche andere, dem Fachmann bekannte Varianten denkbar.

Nach dem in Kontakt bringen der Rauchgase mit der wäßrigen Ammoniaklösung wird die dabei erhaltene Reaktionslösung in einer geeigneten Vorrichtung derart erhitzt, dass sich das in der Reaktionslösung enthaltene Ammoniumcarbonat zersetzt und Kohlendioxid und Ammoniak in die Gasphase übergehen, das Ammoniumsulfat jedoch unzersetzt in der Reaktionslösung verbleibt. Dazu wird die Reaktionslösung bei Normaldruck auf mehr als 58°C erhitzt - bei dieser Temperatur beginnt die Zersetzung von Ammoniumcarbonat. Zur Beschleunigung der Zersetzung ist es möglich, die Zersetzung bei einem verminderten Druck oder in Gegenwart eines entsprechenden Katalysators durchzuführen.

Mit diesem Verfahrensschritt ist es also möglich, zuvor aus den Rauchgasen durch Umsetzung zu Ammoniumcarbonat extrahiertes Kohlendioxid gezielt wieder aus der Reaktionslösung zu entfernen. Nach dem Erhitzen weist die Reaktionslösung primär gelöstes Ammoniumsulfat auf. Das durch das Erhitzen der Reaktionslösung erhaltene Kohlendioxid und Ammoniak werden anschließend in einer geeigneten Vorrichtung gemäß einem aus dem Stand der Technik bekannten Verfahren zu Harnstoff umgesetzt.

Die technische Herstellung von Harnstoff aus Ammoniak und Kohlendioxid ist seit längerem bekannt. Zur Bereitstellung des Eduktes Kohlendioxid wird dazu üblicherweise Erdgas verbrannt. Diese Vorgehensweise zur Bereitstellung des Eduktes Kohlendioxid weist jedoch den Nachteil auf, dass ein kostbarer Energieträger (Erdgas) verwendet wird, um ein vergleichsweise minderwertiges Produkt (Kohlendioxid) zu erhalten. Die vorliegende Erfindung geht hier einen vollständig anderen Weg - das in Rauchgasen von Verbrennungsanlagen reichlich vorhandene Abfallprodukt Kohlendioxid wird mit einer wäßrigen Ammoniaklösung aus den Rauchgasen entfernt und so nutzbar gemacht. Es ist also nicht notwendig, einen wertvollen Energieträger zur Bereitstellung eines Eduktes der Harnstoffsynthese zu verbrennen, sondern es werden Abgase von Verbrennungsanlagen für diesen Zweck genutzt. Vorteilhafterweise bewirkt diese Vermeidung der Verbrennung eines kostbaren Energieträgers gleichzeitig bzw. zusätzlich eine Reduktion der CO₂-Immission, da zumindest ein Teil des Kohlendioxidgehalts der Rauchgase in Form von Harnstoff chemisch gebunden wird.

Mit dem vorliegenden Verfahren ist es also möglich, einen erheblichen Teil des in Rauchgasen vorhandenen "Abfallprodukts" Kohlendioxids in einen Wertstoff, nämlich Harnstoff, umzuwandeln. Harnstoff ist ein für die chemische Großindustrie wichtiger Grundstoff, der in großen Mengen benötigt wird. Die Erfindung erlaubt es also, die Immission von Kohlendioxid erheblich zu vermindern, wobei dies unter Umwandlung eines Abfallproduktes in einen Wertstoff geschieht. Dieser Wertstoff lässt sich veräußern, so dass das erfindungsgemäße Verfahren somit im Ganzen kostengünstig zu führen ist. Erst dieser Synergieeffekt, zum einen die Verminderung der CO₂-Immission und zum anderen die Umwandlung eines Abfallstoffes in einen Wertstoff, ermöglicht ein kostengünstiges Verfahren, das sich im großtechnischen Maßstab anwenden lässt.

Bei einer vorteilhaften Ausführungsform des erfindungsgemäßen Verfahrens umfaßt Schritt a) ferner ein Entschwefeln der Rauchgase. Dies ist insbesondere dann vorteilhaft, wenn die Rauchgase der Verbrennungsanlagen hohe SO₂-Konzentrationen aufweisen. Durch den zusätzlichen Entschwefelungsschritt ist es möglich, die Schwefeldioxid-Konzentration des Rauchgases gezielt einzustellen und damit die Konzentration von Ammoniumsulfat in der Reaktionslösung zu beeinflussen. Das erfindungsgemäße Verfahren ist dabei nicht auf ein bestimmtes Entschwefelungsverfahren beschränkt - sämtliche aus dem Stand der Technik bekannte Verfahren können eingesetzt werden.

Moderne Verbrennungsanlagen erzeugen beträchtliche Mengen Kohlendioxid. Für die Umsetzung zumindest eines Teils des Kohlendioxids sind entsprechend große Mengen Ammoniak notwendig. Vorteilhafterweise wird das für die wäßrige Ammoniaklösung notwendige Ammoniak vor Ort durch eine Ammoniaksynthese hergestellt. Auf diese Weise wird der aufwendige und kostenintensive Transport sowie die Lagerung des Ammoniaks vermieden und es steht stets eine ausreichende Menge Ammoniak zur Verfügung.

Bei dem erfindungsgemäßen Verfahren entsteht in der Reaktionslösung neben Ammoniumcarbonat Ammoniumsulfat. Ammoniumsulfat ist ein wichtiger Düngemittelzusatzstoff und wird in der chemischen Industrie unter anderem als Eiweißfällungsmittel, als Flotationsmittel zur Kunstharzherstellung und zur Herstellung von Feuerlöschpulver und Flammschutzmitteln verwendet. Vorteilhafterweise wird daher aus der Reaktionslösung nach dem Erhitzen derart, dass sich Ammoniumcarbonat zersetzt, Ammoniumsulfat abgeschieden. Auf eine besonders einfache und daher vorteilhafte weise kann das Abscheiden des Ammoniumsulfats mit einer Sprühtrocknung durchgeführt werden.

Bei der Umsetzung von Schwefeldioxid zu Ammoniumsulfat muß der Schwefel oxidiert werden. Vorzugsweise wird als Oxidationsmittel Luft verwendet. Diese ist in beliebiger Menge verfügbar und die Handhabung bereitet keinerlei Probleme.

Bei bekannten Verfahren zur Herstellung von Harnstoff wird ein NH₃/CO₂-Eduktverhältnis von 2,5 - 4 verwendet - NH₃ wird also in einem Überschuß gegenüber CO₂ verwendet. Zur Kostenreduktion und Minimierung der erforderlichen Ammoniakmenge ist es daher vorteilhaft, dass das überschüssige Ammoniak für die Harnstoffsynthese oder zur Herstellung der wäßrigen Ammoniaklösung rezirkuliert wird.

Eine Vorrichtung zum Reinigen von Rauchgasen aus einer Verbrennungsanlage weist Einrichtungen zum Entsticken und Entstauben der Rauchgase auf. Diese schließen sich direkt an die Verbrennung an und können nach im Stand der Technik bekannten Verfahren betrieben werden. Die Schaltung der Einrichtungen zum Entsticken und Entstauben ist beliebig.

Den Einrichtungen zum Entsticken und Entstauben ist eine Wascheinrichtung nachgeschaltet, in der zumindest ein Teil der Rauchgase in der Gegenwart eines Oxidationsmittels mit einer wäßrigen Ammoniaklösung in Kontakt gebracht wird, wobei sich eine Reaktionslösung bildet, die zumindest Ammoniumcarbonat aufweist. Neben Ammoniumcarbonat enthält die Reaktionslösung ferner Ammoniumsulfat, das sich durch Umsetzung von Ammoniak und Schwefeldioxid in der Gegenwart eines Oxidationsmittels bildet.

Der Wascheinrichtung ist ein Stripper nachgeschaltet, in dem die Reaktionslösung derart behandelt wird, dass sich das in der Reaktionslösung enthaltene Ammoniumcarbonat in Ammoniak und Kohlendioxid zersetzen. Um eine entsprechende Zersetzung zu erreichen, muß man die Reaktionslösung bei Normaldruck auf über 58°C erhitzen. Die Zersetzung von Ammoniumcarbonat kann durch zahlreiche Maßnahmen, wie beispielsweise eine Druckverminderung, gefördert werden.

Dem Stripper ist eine Harnstoffanlage nachgeschaltet, in der Ammoniak und Kohlendioxid zu Harnstoff umgesetzt werden. Diese Umsetzung kann nach im Stand der Technik bekannten Verfahren zur Harnstoffsynthese erfolgen. Im nachfolgenden wird das erfindungsgemäße Verfahren bzw. die Vorrichtung anhand eines bevorzugten Ausführungsbeispiels im Zusammenhang mit der beigefügten Zeichnung beschrieben. Die Zeichnung zeigt in:

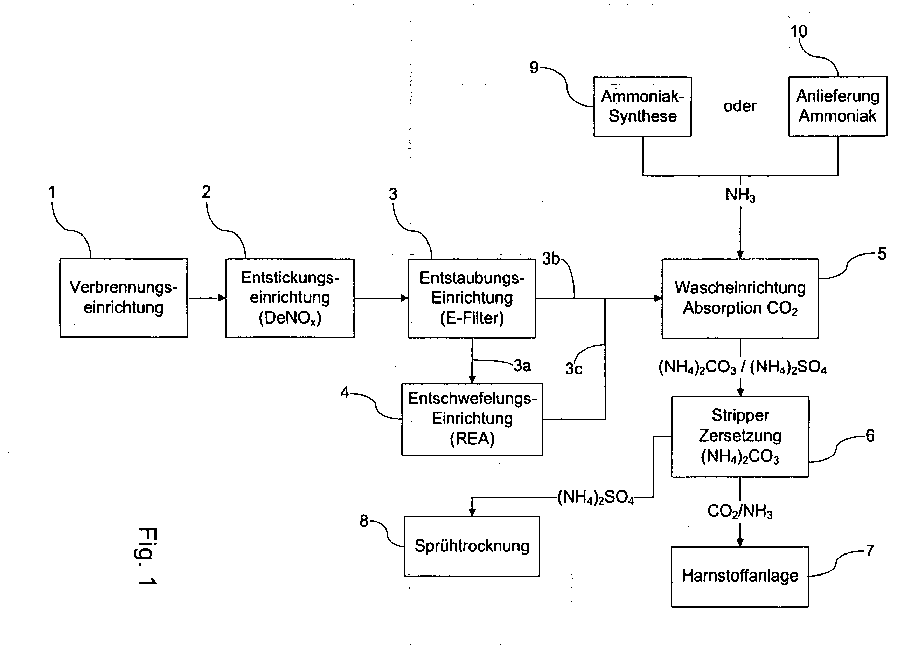

Figur 1 ein Ablaufschema einer bevorzugten Ausführungsform des erfindungsgemäßen Verfahrens bzw. der Vorrichtung.

Die Vorrichtung nach Figur 1 weist eine Verbrennungseinrichtung (1) auf. Das bei der Verbrennung entstehende, schadstoffbelastete Rauchgas gelangt über eine Leitung in eine Entstickungseinrichtung (2), in welcher Stickoxide aus dem Rauchgas entfernt werden. Bei der Vorrichtung gemäß Figur 1 ist der Entstickungseinrichtung (2) eine Entstaubungseinrichtung (3) nachgeschaltet, in welcher feste Bestandteile (Asche) aus dem Rauchgasstrom abgeschieden werden.

Von der Entstaubungseinrichtung (3) führen Leitungen (3a, 3b) zu einer Entschwefelungseinrichtung (4) und einer Wascheinrichtung (5), wobei die Entschwefelungseinrichtung (3) wiederum über eine Leitung (3c) mit der Wascheinrichtung verbunden ist. Bei einer derartigen Anordnung der einzelnen Einrichtungen ist es möglich, einen beliebigen Volumenanteil des Rauchgasstromes durch die Entschwefelungsanlage zu leiten. Für den Fall, dass der Rauchgasstrom lediglich einen geringen Anteil Schwefeldioxid aufweist, kann auf die Leitung durch die separate Entschwefelungsanlage verzichtet werden. Vorhandene Spuren Schwefeldioxid können in der Wascheinrichtung (5) entfernt werden.

Der Entstaubungseinrichtung (3) bzw. der Entschwefelungseinrichtung (4) nachgeschalter ist eine Wascheinrichtung (5), in welcher zumindest ein Teil der Rauchgase mit einer wäßrigen Ammoniaklösung in Kontakt gebracht wird. Bei dieser Inkontaktbringung setzt sich in den Rauchgasen enthaltenes Kohlendioxid zu Ammoniumcarbonat um, wobei eine dieses enthaltene Reaktionslösung entsteht. Bei der Inkontaktbringung setzt sich ferner in den Rauchgasen enthaltenes Schwefeldioxid in der Gegenwart von einem Oxidationsmittel zu Ammoniumsulfat um, wobei üblicherweise Luftsauerstoff als Oxidationsmittel verwendet wird. Für den Fall, dass der Luftsauerstoffgehalt der Rauchgase zu gering ist, kann zusätzlicher Luftsauerstoff zugeführt werden. Dieser kann dem Rauchgasstrom vor der Wascheinrichtung zugeführt werden, oder unmittelbar vor oder bei der Inkontaktbringung mit der Ammoniaklösung. Nach der Umsetzung der Rauchgase mit der Ammoniaklösung werden die verbliebenen Rauchgase abgeführt und nach ggf. weiteren Reinigungsschritten an die Atmosphäre abgegeben.

Das zur Herstellung der wäßrigen Ammoniaklösung notwendige Ammoniak kann entweder in einer Ammoniaksyntheseanlage (9) vor Ort hergestellt werden, oder angeliefert und aus entsprechenden Vorratstanks (10) der Wascheinrichtung (5) zugeführt werden.

Die sich bei der Inkontaktbringung ausbildende Reaktionslösung wird aus der Wascheinrichtung (5) abgezogen und über eine Leitung einem Stripper (6) zugeführt, in welchem diese Lösung derart behandelt wird, dass sich das in dieser enthaltene Ammoniumcarbonat zersetzt. Diese Behandlung kann beispielsweise eine Erwärmung der Reaktionslösung auf > 58°C sein - oberhalb dieser Temperatur zerfällt das Ammoniumcarbonat in seine Edukte - Ammoniak und Kohlendioxid, die als Reichgasstrom abgezogen werden und über eine Leitung der Harnstoffanlage (7) zugeführt werden. In dieser werden Ammoniak und Kohlendioxid - ggf. unter Zuführung weiteren Ammoniaks - zu Harnstoff umgesetzt. Die Harnstoffanlage (7) selbst kann nach einem beliebigen, aus dem Stand der Technik bekannten Verfahren betrieben werden. Überschüssiges Ammoniak wird rezirkuliert und kann entweder zur Herstellung der wäßrigen Ammoniaklösung genutzt werden oder direkt bei der Harnstoffsynthese wiederverwendet werden.

Die bei der Zersetzung von Ammoniumcarbonat verbleibe Reaktionslösung weist einen von der Schwefeldioxid-Konzentration der in die Wascheinrichtung (5) gelangenden Rauchgase abgängigen Anteil Ammoniumsulfat auf. Um dieses nutzbar zu machen wird die Reaktionslösung aus dem Stripper abgezogen und einer Sprühtrocknungsanlage zugeführt. An die Sprühtrocknungsanlage kann sich ggf. noch eine Anlage zur Aufreinigung des Ammoniumsulfats anschließen.

## Patentansprüche

1. Ein Verfahren zum Reinigen von Rauchgasen aus Verbrennungsanlagen, aufweisend die Schritte:
a) Entstauben und Entsticken der Rauchgase,
b) in Kontakt bringen der Rauchgase mit einer wäßrigen Ammoniaklösung in der Gegenwart eines Oxidationsmittels, wobei sich eine Reaktionslösung bildet, die zumindest Ammoniumcarbonat aufweist,
c) Erhitzen der Reaktionslösung derart, das sich Ammoniumcarbonat zersetzt und Kohlendioxid und Ammoniak in die Gasphase übergehen,
d) Umsetzen des gasförmigen Kohlendioxids und des gasförmigen Ammoniaks zu Harnstoff.

2. Das Verfahren nach Anspruch 1, **dadurch gekennzeichnet, dass** Schritt a) ferner ein Entschwefeln umfaßt.

3. Das Verfahren nach Anspruch 1 oder 2, **dadurch gekennzeichnet, dass** das für die wäßrige Ammoniaklösung notwendige Ammoniak vor Ort durch eine Ammoniaksynthese hergestellt wird.

4. Das Verfahren nach einem der Ansprüche 1 bis 3, **dadurch gekennzeichnet, dass** aus der Reaktionslösung von Schritt c) nach dem Erhitzen derart, das sich Ammoniumcarbonat zersetzt, Ammoniumsulfat abgeschieden wird.

5. Das Verfahren nach Anspruch 4, **dadurch gekennzeichnet, dass** das Abscheiden des Ammoniumsulfats mit einer Sprühtrocknung durchgeführt wird.

6. Das Verfahren nach einem der Ansprüche 1 bis 5, **dadurch gekennzeichnet, dass** als Oxidationsmittel Luft verwendet wird.

7. Das Verfahren nach einem der Ansprüche 1 bis 6, **dadurch gekennzeichnet, dass** überschüssiges Ammoniak aus Schritt d) zur Harnstoffsynthese oder zur Herstellung der wäßrigen Ammoniaklösung rezirkuliert wird.

## Claims

1. A method of cleaning flue gases of incineration plants, comprising the steps:
a) removal of dust and nitrogen oxide from the flue gases,
b) bringing the flue gases into contact with an aqueous ammonia solution in the presence of an oxidising agent, wherein a reaction solution comprising at least ammonium carbonate forms,
c) heating the reaction solution so that ammonium carbonate decomposes and carbon dioxide and ammonia transform into the gas phase and
d) converting the gaseous carbon dioxide and gaseous ammonia into urea.

2. The method according to claim 1, **characterised in that** step a) further comprises removal of sulphur.

3. The method according to claim 1 or 2, **characterised in that** the ammonia necessary for the aqueous ammonia solution is produced on site by ammonia synthesis.

4. The method according to any one of claims 1 to 3, **characterised in that** ammonium sulphate is separated from the reaction solution of step c) after the heating so that ammonium carbonate decomposes.

5. The method according to claim 4, **characterised in that** the separation of the ammonium sulphate is carried by a spray drying.

6. The method according to any one of claims 1 to 5, **characterised in that** air is used as oxidising agent.

7. The method according to any one of claims 1 to 6, **characterised in that** excess ammonia from step d) is recirculated for the urea synthesis or for the production of the aqueous ammonia solution.

## Revendications

1. Procédé d'épuration des gaz de combustion provenant d'incinérateurs, comprenant les étapes:
a) dépoussiérage et dénitratation des gaz de combustion,
b) mise en contact des gaz de combustion avec une solution aqueuse d'ammoniac en présence d'un agent oxydant, moyennant quoi il se forme une solution de réaction qui comporte au moins du carbonate d'ammonium,
c) chauffage de la solution de réaction de telle sorte que le carbonate d'ammonium se décompose et que le dioxyde de carbone et l'ammoniac passent dans la phase gazeuse,
d) conversion du dioxyde de carbone gazeux et de l'ammoniac gazeux en urée.

2. Procédé selon la revendication 1, **caractérisé en ce que** l'étape a) comporte en outre une désulfuration.

3. Procédé selon la revendication 1 ou 2, **caractérisé en ce que** l'ammoniac nécessaire pour la solution aqueuse d'ammoniac est produit sur site par une synthèse ammoniacale.

4. Procédé selon l'une quelconque des revendications 1 à 3, **caractérisé en ce qu'**après le chauffage effectué de telle sorte que le carbonate d'ammonium se décompose, du sulfate d'ammonium est séparé de la solution de réaction de l'étape c).

5. Procédé selon la revendication 4, **caractérisé en ce que** la séparation du sulfate d'ammonium est effectuée par un séchage par pulvérisation.

6. Procédé selon l'une quelconque des revendications 1 à 5, **caractérisé en ce que** l'air est utilisé comme agent oxydant.

7. Procédé selon l'une quelconque des revendications 1 à 6, **caractérisé en ce que** l'ammoniac en excédent de l'étape d) est remis en circulation pour la synthèse de l'urée ou pour la préparation de la solution aqueuse d'ammoniac.
